# EUROPEAN PATENT APPLICATION

(11) **EP 2 537 552 A1**
(43) Date of publication of application: **26.12.2012**
(21) Application number: 11855249.6
(22) Date of filing: 07.09.2011
(51) Int. Cl.: A61M 39/00, A61M 5/14, A61M 27/00, A61F 13/00

(54) **APPARATUS FOR FIXING A MEDICAL PIPE**

(30) Priority: 15.03.2011 KR 20110022845
(71) Applicant: Lee, Keun Ho, Gyeunggi-do 420-030 (KR)
(72) Inventor: Lee, Keun Ho, Gyeunggi-do 420-030 (KR)
(74) Representative: Gervasi, Gemma
(86) International application number: PCT/KR2011/006605
(87) International publication number: WO 2012/124866

(57) **Abstract**

Disclosed is a medical tube fixing device, and more particularly a medical tube fixing device, which may ensure rapid coupling with a medical tube with minimal movement of the medical tube, and which includes a fixing member attached to a human body, the fixing member being flexibly operated in response to movement of the medical tube, thereby preventing deterioration in adhesive strength thereof. With provision of the medical tube fixing device, it is possible to rapidly fix or separate the medical tube to or from a desired portion of the human body, and to minimize movement of the fixing member caused during movement of the medical tube, thereby preventing deterioration in adhesive strength of the fixing member.

## Description

### [Technical Field]

The present invention relates to a medical tube fixing device, and more particularly to a medical tube fixing device, which may ensure rapid coupling with a medical tube with minimal movement of the medical tube, and which includes a fixing member attached to a human body, the fixing member being flexibly operated in response to movement of the medical tube, thereby preventing deterioration in adhesive strength thereof.

### [Background Art]

As known in general, drainage tubes include a catheter that is used to discharge bile, abscess, etc. from the human organs, such as the liver, the stomach, etc., to the outside of the human body, or an indwelling catheter inserted into the bladder to discharge urine. In use of such a drainage tube, one end is inserted into the human body and the other end protrudes from the human body.

A conventional method for fixing the inserted drainage tube, comprising binding a portion of the drainage tube protruding out of the human body using a surgical thread fixed to the human body and covering the bound portion with dressings, has been used.

However, the above described conventional method has a problem in that firmly fixing the drainage tube using the surgical thread is impossible, which causes the drainage tube to be pushed little by little outward of the human body when a patient moves his/her body or upon exchange of dressings.

Another problem of the conventional method is that the patient or a nurse cannot easily recognize that the drainage tube is pushed little by little outward of the human body.

Meanwhile, although it is impossible to firmly fix the drainage tube using the surgical thread, the fixing operation of the drainage tube using the surgical thread requires a great time, and release of the drainage tube also takes a long time. In addition, the above described and other conventional technologies are unfavorable for patients because fixing the drainage tube to the human body requires frequent movement of the drainage tube.

### [Disclosure]

### [Technical Problem]

The present invention is devised in consideration of the above described problems of the related art, and it is an aspect of the present invention to provide a medical tube fixing device, which may ensure rapid coupling with a medical tube with minimal movement of the medical tube, and which includes a fixing member attached to a human body, the fixing member being flexibly operated in response to movement of the medical tube, thereby preventing deterioration in adhesive strength thereof.

### [Technical Solution]

In accordance with one aspect of the present invention, a medical tube fixing device includes a fixing member configured to be attached to a human body and which is provided at a predetermined center position of an upper surface thereof with a coupling band, and a tube fastening loop having an opening at one side thereof and which consists of a band binding portion, to which the coupling band is bound, and a tube coupling portion, to which a medical tube is coupled, both the band binding portion and the tube coupling portion extending from the opening in opposite directions.

The fixing member may include an adhesive sheet attached to the skin of the human body, a fixing piece secured to a predetermined center position of the adhesive sheet to fix the coupling band to the adhesive sheet, an upper protective sheet attached to an upper surface of the adhesive sheet, and a release paper attached to a lower adhesive surface of the adhesive sheet.

The tube fastening loop may have reinforcing ribs protruding from edges of both lateral surfaces thereof.

The band binding portion of the tube fastening loop may define a lower portion of a loop body and may be provided with a band coupling hole, and the tube coupling portion may define an upper portion of the loop body and may be provided with a tube coupling hole.

A first coupling protrusion and a second coupling protrusion may be respectively formed at opposite sides of the opening, and a distance between the first coupling protrusion and the second coupling protrusion may be less than a diameter of the tube.

The coupling band may be a flexible band.

### [Advantageous Effects]

With provision of a medical tube fixing device according to the present invention, it is possible to rapidly fix or separate a medical tube to or from a desired portion of a human body, and to minimize movement of a fixing member caused during movement of the medical tube, thereby preventing deterioration in adhesive strength of the fixing member.

### [Description of Drawings]

FIG. 1 is an exploded perspective view illustrating a medical tube fixing device according to an embodiment of the present invention,
FIG. 2 is a perspective view illustrating a tube fastening loop included in the medial tube fixing device according to the embodiment of the present invention,
FIG. 3 is a front view illustrating the tube fastening loop included in the medial tube fixing device according to the embodiment of the present invention, and
FIG. 4 is a perspective view illustrating a state in which the medial tube fixing device according to the embodiment of the present invention is coupled to a medical tube.

### * Description of Reference Numerals

| | | | |
|---|---|---|---|
| 2: | medical tube fixing device | 4: | fixing member |
| 6: | adhesive sheet | 12: | coupling band |
| 20: | tube fastening loop | 30: | tube coupling portion |
| 40: | band binding portion | | |

### [Best Mode]

Reference will now be made in detail to the exemplary embodiments of the present invention, examples of which are illustrated in the accompanying drawings.

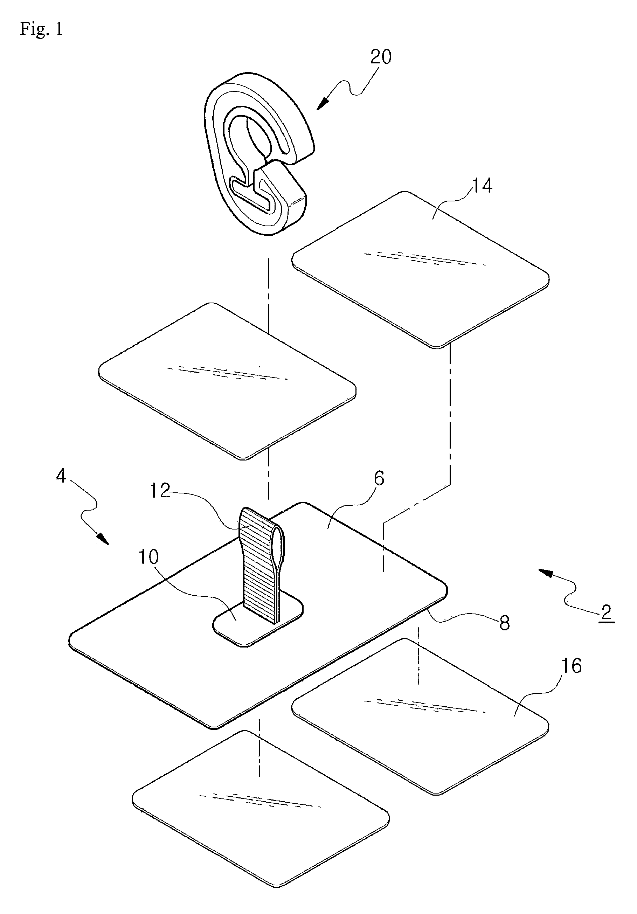

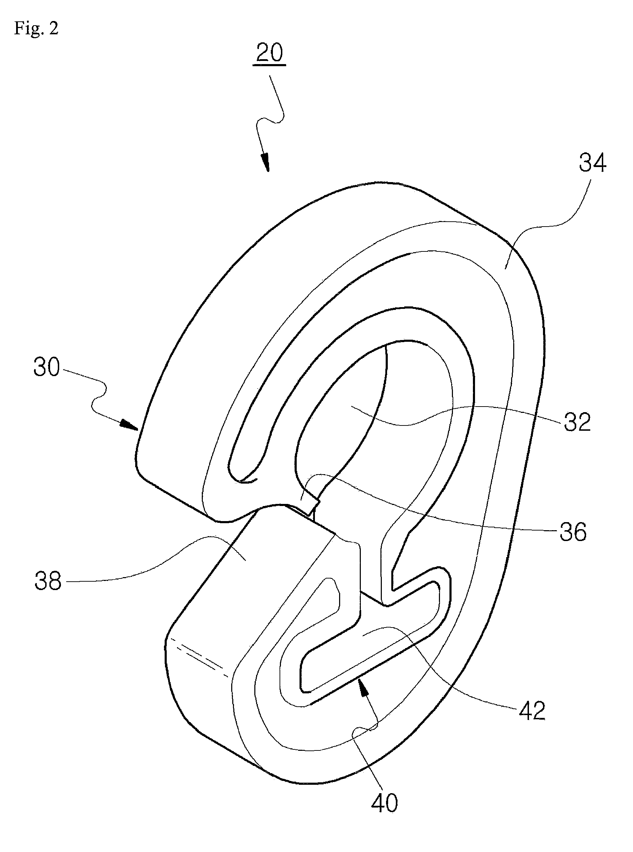

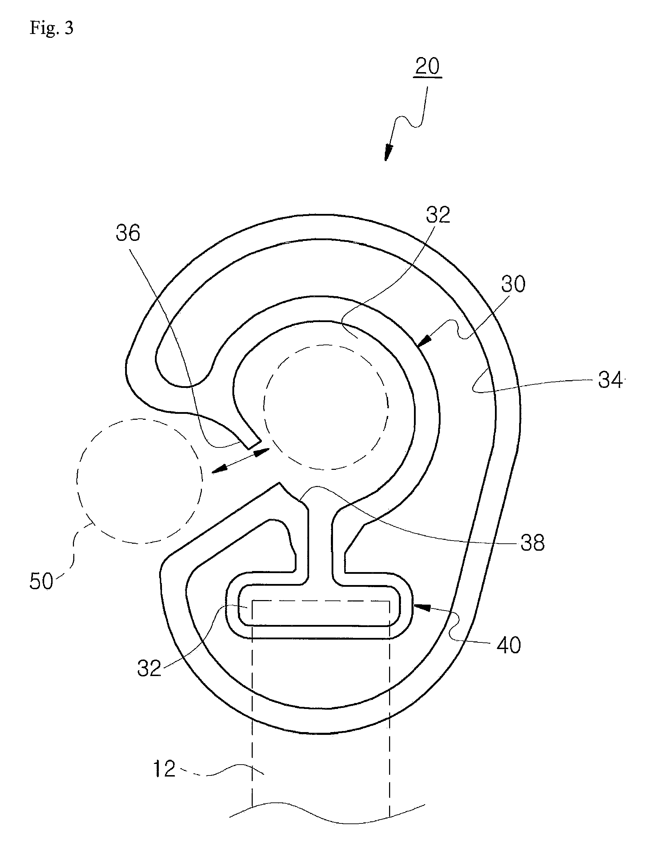

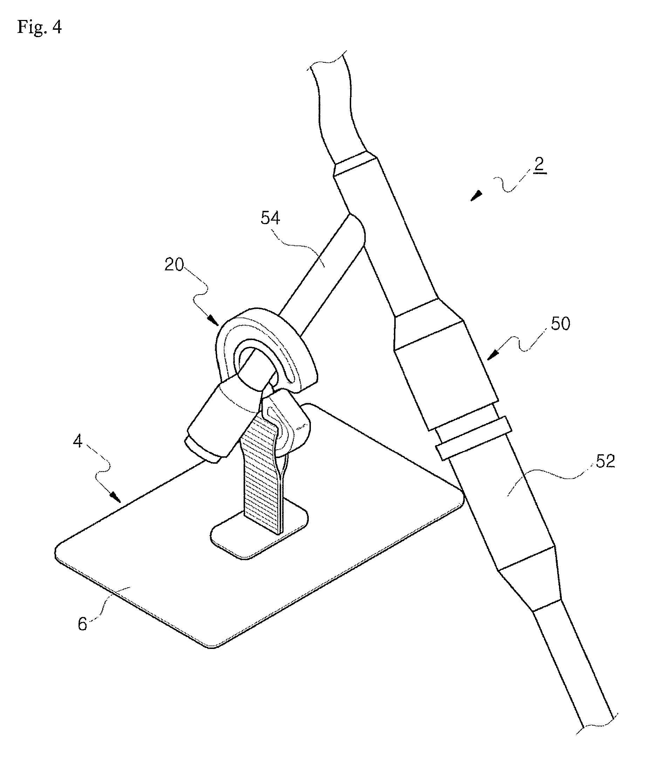

FIG. 1 is an exploded perspective view illustrating a medical tube fixing device according to an embodiment of the present invention, FIG. 2 is a perspective view illustrating a tube fastening loop included in the medial tube fixing device according to the embodiment of the present invention, FIG. 3 is a front view illustrating the tube fastening loop included in the medial tube fixing device according to the embodiment of the present invention, and FIG. 4 is a perspective view illustrating a state in which the medial tube fixing device according to the embodiment of the present invention is coupled to a medical tube.

As shown in the drawings, the medical tube fixing device 2 according to the embodiment of the present invention is configured to ensure rapid coupling with a medical tube with minimal movement of the medical tube and includes a fixing member attached to a human body, the fixing member being flexibly operated in response to movement of the medical tube, thereby preventing deterioration in adhesive strength thereof.

More specifically, the medical tube fixing device 2 according to the embodiment of the present invention includes a fixing member 4 which is configured to be attached to a human body and is provided at a predetermined center position of an upper surface thereof with a coupling band 12, and a tube fastening loop 20 which has an opening at one side thereof and consists of a band binding portion 40, to which the coupling band 12 is bound, and a tube coupling portion 30, to which a medical tube 50 is coupled, both the band binding portion and the tube coupling portion extending from the opening in opposite directions.

The fixing member 4 serves to assist the medical tube 50 in being fixed to the human body while preventing movement of the medical tube beyond a predetermined range. Preferably, the fixing member 4 is configured to prevent adhesive strength thereof from being deteriorated by inevitable movement of the medical tube 50. This will be described in detail hereinafter.

The fixing member 4 includes an adhesive sheet 6 having a lower surface to which an adhesive is applied to allow the fixing member to be attached to the skin of the human body, a fixing piece 10 secured to a predetermined center position of the adhesive sheet 6 to fix the coupling band 12 to the adhesive sheet 6, an upper protective sheet 14 attached to an upper surface of the adhesive sheet 6, and a release paper 16 attached to the lower adhesive surface 8 of the adhesive sheet 6.

Preferably, since the fixing piece 10 must have a predetermined strength sufficient to exhibit no softness and must be attached to the upper surface of the adhesive sheet 6, an adhesive is applied to a lower surface of the fixing piece 10 to allow the fixing piece to be attached to the center of the adhesive sheet 6.

The upper protective sheet 14 is divided into two sheets on the basis of the fixing piece 10, and facing ends of the two sheets overlap each other by a constant length.

Similarly, the release paper 16 is divided into two pieces of paper on the basis of the adhesive sheet 6, and facing ends of the two pieces of paper overlap each other to ensure that the release paper 16 is easily released from the adhesive sheet 6.

More specifically, the tube fastening loop 20 included in the medical tube fixing device according to the embodiment of the present invention has reinforcing ribs 34 protruding from edges of both lateral surfaces thereof, thereby achieving high strength despite a small area thereof. The tube fastening loop is formed of synthetic resin.

The band binding portion 40 constituting the tube fastening loop 20 defines a lower portion of a loop body and is provided with a band coupling hole 42. The tube coupling portion 30 defines an upper portion of the loop body and is provided with a tube coupling hole 32.

In this case, the band coupling hole 42 and the tube coupling hole 32 are cut to communicate with each other and also to communicate with the opening of the loop, which enables primary insertion of the coupling band 12 or the medical tube 50 through the opening.

Once primarily inserted through the opening, the coupling band 12 or the medical tube 50 is coupled into the band coupling hole 42 or the tube coupling hole 32. A first coupling protrusion 36 and a second coupling protrusion 38 are respectively formed at opposite sides of the opening formed in one side of the tube fastening loop 20. A distance between the first coupling protrusion 36 and the second coupling protrusion 38 is less than a diameter of the tube 50.

Accordingly, to couple the medical tube 50 into the tube coupling hole 32, it is necessary to push the medical tube 50 into the tube fastening loop in a state in which the medical tube comes into close contact with the opening. Thereby, the medical tube 50 acts to move the first coupling protrusion 36 and the second coupling protrusion 38 away from each other, causing the medical tube to be coupled into the tube coupling hole 32. After completion of the coupling operation, the first coupling protrusion 36 and the second coupling protrusion 38 are returned to original positions thereof, thereby preventing the medical tube 50 from being unexpectedly separated from the tube coupling hole 32.

To this end, any one of the first coupling protrusion 36 and the second coupling protrusion 38 must be more flexible than the other. However, since adoption of two different kinds of materials complicates fabrication and is less economical, one of the first coupling protrusion 36 and the second coupling protrusion 38 may be configured to have a smaller thickness than the other. In the present invention, the thickness of the first coupling protrusion 36 may be less than the thickness of the second coupling protrusion 38, to ensure easy attachment/detachment of the medical tube 50.

Meanwhile, the band coupling hole 42 has a narrow entrance, and an inner circumference of the band coupling hole is linearly shaped to easily keep the coupling band 12 in a coupled state. Accordingly, once the coupling band 12 is coupled into the band coupling hole 42, as shown by a dotted line of FIG. 3, the coupling band 12 is firmly seated and coupled to the inner circumference of the band coupling hole 42.

As described above, the fixing member 4 serves to assist the medical tube 50 in being fixed to the human body while preventing movement of the medical tube beyond a predetermined range. Preferably, the fixing member 4 is configured to prevent adhesive strength thereof from being deteriorated by movement of the medical tube even if the medical tube 50 is inevitably moved.

More specifically, in the case in which the medical tube 50 and the fixing member 4 are connected to each other, and the medical tube 50 must be inevitably moved in a state in which the adhesive surface 8 of the fixing member 4 is attached to the human body, it is advantageous that the fixing member 4 not be moved or be moved only within a small range.

If the fixing member 4 is moved equally to movement of the medical tube 50, this causes the adhesive sheet 6 of the fixing member 4 to be separated from the human body, resulting in deterioration in adhesive strength of the fixing member. In this case, the fixing member 4 cannot correctly function and must be replaced.

To solve the above described problem, in the present invention, the coupling band 12 is formed of a flexible band to prevent movement of the fixing member 4 or to reduce the movement range of the fixing member during movement of the medical tube 50.

In the medical tube fixing device 2 according to the embodiment of the present invention, first, the fixing member 4 is attached to the human body so as to be primarily fixed, and then the tube fastening loop 20 is fitted into the coupling band 12 of the fixing member 4 so as to be coupled to the fixing member 4. In the coupled state, as shown in FIG. 4, if the medical tube, such as a drainage tube 52 or a saline tube 54, is fitted and pushed into the opening formed in one side of the tube fastening loop 20, the medical tube 50 may be easily secured to a desired position of the human body.

Meanwhile, the medical tube fixing device according to the embodiment of the present invention is not limited to the above described embodiment, and various modifications are possible without departing from the scope and spirit of the invention.

## Claims

1. A medical tube fixing device comprising:
a fixing member configured to be attached to a human body and which is provided at a predetermined center position of an upper surface thereof with a coupling band; and
a tube fastening loop having an opening at one side thereof and which consists of a band binding portion, to which the coupling band is bound, and a tube coupling portion, to which a medical tube is coupled, both the band binding portion and the tube coupling portion extending from the opening in opposite directions.

2. The medical tube fixing device according to claim 1, wherein the fixing member includes:
an adhesive sheet attached to the skin of the human body;
a fixing piece secured to a predetermined center position of the adhesive sheet to fix the coupling band to the adhesive sheet;
an upper protective sheet attached to an upper surface of the adhesive sheet; and
a release paper attached to a lower adhesive surface of the adhesive sheet.

3. The medical tube fixing device according to claim 1, wherein the tube fastening loop has reinforcing ribs protruding from edges of both lateral surfaces thereof.

4. The medical tube fixing device according to claim 1, wherein the band binding portion of the tube fastening loop defines a lower portion of a loop body and is provided with a band coupling hole, and the tube coupling portion defines an upper portion of the loop body and is provided with a tube coupling hole.

5. The medical tube fixing device according to claim 1, wherein a first coupling protrusion and a second coupling protrusion are respectively formed at opposite sides of the opening, and a distance between the first coupling protrusion and the second coupling protrusion is less than a diameter of the tube.

6. The medical tube fixing device according to claim 2, wherein the coupling band is a flexible band.
